# EUROPÄISCHE PATENTSCHRIFT

(11) **EP 2 475 318 B1**
(45) Veröffentlichungstag und Bekanntmachung des Hinweises auf die Patenterteilung: **09.01.2019**
(21) Anmeldenummer: 10759808.8
(22) Anmeldetag: 07.09.2010
(51) Int. Cl.: A61B 18/00, A61B 18/04, H05H 1/46, H05H 1/24

(54) **MULTIFUNKTIONSELEMENT ZUR VERHINDERUNG DER KARBONISIERUNG VON GEWEBE MITTELS EINES MULTIFUNKTIONSELEMENTS**
MULTI-FUNCTIONAL ELEMENT FOR HINDERING THE CARBONISATION OF TISSUE BY MEANS OF A MULTI-FUNCTIONAL ELEMENT
ÉLÉMENT MULTIFONCTIONNEL EMPÊCHANT LA CARBONISATION DE TISSU AU MOYEN D'UN ÉLÉMENT MULTIFONCTIONNEL

(30) Priorität: 11.09.2009 DE 102009041167
(43) Veröffentlichungstag der Anmeldung: 18.07.2012
(73) Patentinhaber: Erbe Elektromedizin GmbH, 72072 Tübingen (DE)
(72) Erfinder: FISCHER, Klaus, 72202 Nagold (DE); NEUGEBAUER, Alexander, 72116 Mössingen (DE); ENDERLE, Markus, 72070 Tübingen (DE); ZENKER, Matthias, 72074 Tübingen (DE)
(74) Vertreter: Rüger Abel
(86) Internationale Anmeldenummer: PCT/EP2010/005484
(87) Internationale Veröffentlichungsnummer: WO 2011/029572

(56) Entgegenhaltungen:
- EP-A1- 1 829 492
- EP-A1- 2 160 081
- EP-A2- 0 740 926
- WO-A1-03/084294
- WO-A1-2006/119892
- WO-A1-2008/090004
- WO-A1-2009/146432
- WO-A2-2007/006517
- US-A1- 2004 116 918
- US-A1- 2007 029 500
- US-A1- 2009 039 790
- US-B1- 6 348 051
- US-B1- 6 582 427

## Beschreibung

Die Erfindung betrifft ein Multifunktionselement nach dem Oberbegriff des Anspruchs 1, welches zur Durchführung von mindestens zwei chirurgisch/therapeutischen Eingriffen geeignet ist
Die Hochfrequenzchirurgie, der auch die Plasma-Koagulation als Teilgebiet zuzuordnen ist, wird seit vielen Jahren sowohl in der Human- als auch in der Veterinärmedizin eingesetzt, um biologisches Gewebe zu koagulieren und/oder zu schneiden. Dabei wird mit Hilfe geeigneter elektrochirurgischer Instrumente hochfrequenter Strom durch das zu behandelnde Gewebe geleitet, so dass sich dieses aufgrund Eiweißkoagulation und Dehydration verändert. Es lassen sich somit aufgrund eines Koagulationsprozesses Gefäße verschließen und Blutungen stillen. Ein auf den Koagulationsprozess folgender Schneidprozess ermöglicht dann die vollständige Trennung bereits koagulierten Gewebes.
Die Plasma-Koagulation ermöglicht ein berührungsfreies Koagulieren von Gewebe und dient der effektiven Blutstillung und Gewebedevitalisierung. Bei dieser Koagulationsart wird inertes Arbeitsgas, z.B. Argon, über Gaszuführungseinrichtungen von einem Plasma-Koagulations-Instrument an das zu behandelnde Gewebe geleitet. Mit Hilfe des Arbeitsgases kann dann ein "Plasmastrahl" zwischen einer Elektrode an einem distalen Ende der Gaszuführungseinrichtung, beispielsweise einer Sonde, und dem Gewebe erzeugt werden. Der HF-Strom lässt sich dann an dem zu behandelnden Gewebe applizieren, ohne dass das elektrochirurgische Instrument mit dem Gewebe in Kontakt kommt. Ein Verkleben des Gewebes mit den Instrumenten wird somit vermieden. Weiterhin soll bei einer Resektion von Gewebe, insbesondere von Tumor-Gewebe im Gastrointestinaltrakt, welches auf die Mu-kosa begrenzt ist, möglichst in einer Sitzung und möglichst vollständig ektomiert werden können. Um auch großflächige Tumore mit einem Durchmesser von mehr als acht Zentimetern in einer Sitzung und möglichst vollständig ektomieren zu können, wird beispielsweise in der WO 2006/108480 A1 vorgeschlagen, bei der endoskopischen Mukosaresektion vor der Resektion zuerst die Mukosa durch eine flexible Nadel mit Flüssigkeit zu unterspritzen. Die Nadel wird dabei in der Submukosa platziert. Durch das Eindringen der Flüssigkeit in die Mukosa wird diese von der Muskularis Propria abgelöst, wobei ein Flüssigkeitskissen unter der Mukosa entsteht. Dadurch werden ein Sicherheitsabstand zur Muskularis Propria sowie ein Wärmeschutz gewonnen. Beispielsweise mit einem flexiblen Nadelmesser, insbesondere aber mit einem oben beschriebenen HF-Chirurgieinstrument wird dann die Mukosaresektion durchgeführt.

Bei dem Wasserstrahlchirurgieinstrument gemäß dem Stand der Technik tritt ein gebündelter Wasserstrahl an einem distalen Ende des Instrumentes mit hohem Druck aus und durchdringt die weiche Schleimhaut (Mukosa). In der Submukosa (in der elastischen faserigen Verschiebeschicht) wird die eindringende Flüssigkeit derart aufgefangen, dass ein Flüssigkeitskissen entsteht.

Aus der WO 2008/0909004 A1 ist ein bipolares Instrument sowie ein Verfahren zur elektrochirurgischen Behandlung von Geweben bekannt. Das Instrument umfasst eine Sonde, an deren Ende zwei elektrische Leiter münden. Zwischen diesen kann ein Lichtbogen gezündet werden. Über einen sich durch das Instrument erstreckenden Kanal kann ein geeignetes Gas, z.B. Argon, zugeführt werden, um eine Schutzgasatmosphäre zu bilden.

Die US 6,582,427 B1 offenbart ein Mehrfunktionsinstrument, das einerseits zum Gewebeschneiden und andererseits zur Gewebekoagulation einsetzbar ist. Es weist dazu eine Elektrodenanordnung auf, die den Betriebsarten entsprechend mit Strom beaufschlagbar ist. Das Instrument enthält einen Kanal zum Zuführen von Argon. Außerdem ist ein zusätzlicher Kanal zum Zuführen eines Oxidationsmittels zu dem Argonfluss vorgesehen, um ein Gas-Oxidationsmittel-Gemisch bereitzustellen. Aus diesem wird ein Gas-Oxidationsmittel-Plasma erzeugt. Die Zuführung des Oxidationsmittels soll eine Karbonisierung des Gewebes verhindern. EP0740926 offenbart eine elektrochirurgische Vorrichtung zur Erzeugung eines Lichtbogens, die ein Aerosol von Luft und Wasser benutzt.

Bei den oben beschriebenen Behandlungen kann es darüber hinaus zu inneren Blutungen kommen, welche die Sicht des Operateurs behindern, so dass der OP-Situs freigespült werden muss. Hierzu ist üblicherweise eine geeignete Spülsonde vorgesehen. Nachteilig an den oben beschriebenen Verfahren ist, dass für jedes ein separates Instrument erforderlich ist, womit ein aufwendiger Wechsel des jeweiligen Instruments bei jedem neuen Eingriff verbunden ist. Weiterhin ist eine gewebeschonende Plasma-Koagulation nur mit niedrigen Leistungen und/oder kurzen Applikationszeiten möglich, da bei höheren Leistungen eine Karbonisierung von koaguliertem Gewebe nicht sicher vermeidbar ist. Durch die Karbonisierung kann es zu einer Entzündung des Gewebes und vermehrt zu post-operativen Problemen kommen. Auch eine durch die Karbonisierung auftretende Rauch- und Rauchgasbildung, die darüber hinaus mit einer unangenehmen Geruchsentwicklung einhergeht, wird nicht sicher vermieden. Die Rauchentwicklung führt außerdem zu einer Sichtbehinderung des Operateurs und gilt es daher unbedingt zu vermeiden. Ein weiterer Nachteil ist die Ausbildung von konzentrierten Strompfaden während der Plasma-Koagulation, die zu einer inhomogenen Schädigung des Gewebes führen. Schließlich können während des Eingriffs auftretende Sickerblutungen durch die bekannten Instrumente nur schwer lokalisiert werden.
Der Erfindung liegt daher die Aufgabe zugrunde, ein Multifunktionselement zur Verhinderung der Karbonisierung von Gewebe mittels eines Multifunktionselements zu schaffen, mit dem wenigstens zwei chirurgisch/therapeutische Eingriffe durchführbar sind, wobei das Multifunktionselement eine optimale Behandlung des Patienten und eine verbesserte Handhabung ermöglicht.
Die Aufgabe der vorliegenden Erfindung wird durch ein Multifunktionselement mit den Merkmalen des Patentanspruchs 1 gelöst. Das Multifunktionselement ist zur Durchführung von mindestens zwei chirurgisch/therapeutischen Eingriffen geeignet, beispielsweise zum Unterspritzen und zum Schneiden von Gewebe. Vorzugweise kann es auch Gewebe koagulieren. Es weist eine Karbonisierungsverhinderungsvorrichtung zum Verhindern der Karbonisierung von Gewebe bei einer Plasma-Koagulation mittels eines geeigneten chirurgischen Instruments auf. Das chirurgische Instrument umfasst eine Zuführung für ein Oxidationsmittel, eine Zuführung für ein Gas und eine Elektrode zur Erzeugung eines Plasmas, wobei die Karbonisierungsverhinderungsvorrichtung ein Gas-Oxidationsmittel-Gemisch zur Erzeugung eines Gas-Oxidationsmittel-Plasmas bereitstellt. Das Multifunktionselement ermöglicht somit die Durchführung von mindestens zwei chirurgisch/therapeutischen Eingriffen, insbesondere eine Plasma-Koagulation und eine Wasserapplikation, insbesondere das Unterspritzen von Gewebe, wobei normalerweise für jeden Eingriff ein separates Instrument erforderlich und damit ein Wechsel der jeweiligen Instrumente notwendig ist. Durch den Einsatz einer Karbonisierungsverhinderungsvorrichtung ist es darüber hinaus möglich, eine optimale gewebeschonende Behandlung des Patienten zu gewährleisten, da eine Karbonisierung des Gewebes und die damit verbundenen Nachteile vermieden werden. Weiterhin sorgt die Karbonisierungsverhinderungsvorrichtung für eine Reduzierung von Rauch bzw. Rauchgasentwicklung, wodurch die Sicht des Operateurs wesentlich verbessert wird und auf eine Lüftung des Operationssaals verzichtet werden kann. Das Multifunktionselement kann weiterhin ein flüssiges Medium zu Spülzwecken einsetzen, beispielsweise Wasser, eine physiologische Kochsalzlösung oder dergleichen. Damit können beispielsweise Sickerblutungen besser erkannt und unmittelbar mit der Plasmachirurgie oder der Elektrochirurgie gestoppt werden.

Insgesamt sind durch das hier vorgeschlagene Multifunktionselement eine gewebeschonende Blutstillung und eine gewebeschonende Devitalisierung von Tumorgewebe möglich. Weiterhin ist es besonders vorteilhaft, dass auch dünnwandige und/oder nervsensible Strukturen ohne Weiteres behandelt werden können. Darüber hinaus ist das Multifunktionselement zur adhäsionsarmen Koagulation in der offenen Chirurgie, zur Laparoskopie und zur flexiblen Endoskopie in besonders vorteilhafter Weise einsetzbar. Durch die Karbonisierungsverhinderungsvorrichtung des Multifunktionselements wird darüber hinaus eine Karbonisierung von Gewebe während der Plasma-Koagulation vermindert, so dass verbesserte Wundheilungsvoraussetzungen gegeben sind. Durch die Karbonisierungsverhinderungsvorrichtung sind eine geruchsärmere Koagulation sowie eine Rauch- und rauchgasarme Koagulation von Gewebe möglich. Dadurch, dass mit dem Multifunktionselement nicht nur HF-chirurgische Eingriffe möglich sind, sondern ebenso Wasserapplikationen, wie beispielsweise ein kontaktloses Unterspritzen von Flüssigkeit zur Erzeugung eines Flüssigkeitskissens (nadellose Injektion) in der Submuskosa-Schicht der Schleimhaut zum Zweck des Aufbaus eines Thermoschutzkissens, kann die Gewebeoberfläche mit einer Standard-Plasma-Koagulation behandelt werden, ohne tiefer liegende Gewebeschichten zu zerstören. Gleichzeitig ist es durch das Multifunktionselement möglich, den OP-Situs zur Verbesserung der Sicht beim Auftreten von Blutungen zu spülen, ohne dass hierfür ein separates Instrument notwendig wäre.

Ein Einsatz des Multifunktionselements kann besonders vorteilhaft in der flexiblen Endoskopie erfolgen, denkbar ist es jedoch auch, das Multifunktionselement für die offene Chirurgie und die Laparoskopie einzusetzen. Insbesondere im endoskopischen Einsatz ist die Karbonisierungsverhinderungsvorrichtung des Multifunktionselements von besonderem Vorteil, da ein Instrumentenwechsel nur selten erforderlich ist und die unvermeidliche Sichtverhinderung durch Rauchgasbildung während der Plasmabehandlung nahezu entfällt.

Besonders bevorzugt wird eine Ausführungsform des Multifunktionselements, bei der das für die Karbonisierungsverhinderungsvorrichtung eingesetzte Oxidationsmittel flüssig oder gasförmig ist. Vorzugsweise wird als Oxidationsmittel Wasser und als Gas ein Inertgas, insbesondere Argon eingesetzt. Das Oxidationsmittel kann auch in Form eines Aerosols vorliegen, sodass es also in feine Oxidationsmitteltröpfchen zu einem Oxidationsmittelnebel zerstäubt ist. Durch den Oxidationsmittelnebel werden die spezifische Oberfläche und damit die Wärmeaustauschfläche zwischen dem Oxidationsmittel und dem Trägergas um mehr als das Hundertfache vergrößert, so dass der Verdampfungspunkt der flüssigen Oxidationsmitteltröpfchen erheblich herabgesetzt ist, der Oxidationsmittelnebel also wesentlich schneller verdampft. Dadurch liegt ein erheblicher Anteil des Oxidationsmittels auch als Oxidationsmitteldampf vor. Auf diese Weise kann ein Teil des Oxidationsmittels, nämlich der gasförmig vorliegende Teil zu einem Oxidationsmitteldampfplasma ionisiert werden. Hierbei bildet sich ein reaktives Plasma, welches im Fall von Wasser als Oxidationsmittel Spezies wie H20<+>, H, OH und O Radikale enthält. Durch die Erhöhung der spezifischen Oberfläche kann die Gewebeoberfläche im Übrigen deutlich gekühlt werden, wodurch eine Karbonisierung reduziert wird. Denkbar ist es auch, das Oxidationsmittel vor der Bereitstellung des Gas-Oxidationsmittel-Gemisches mittels eines Verdampfers in seinen gasförmigen Zustand zu überführen. Weiterhin können dem Oxidationsmittel Nanopartikel mit bestimmten Eigenschaften beigemischt werden, die beispielsweise eine therapeutische Wirkung verstärken oder beschleunigen oder auch Nebenwirkungen verringern können. Denkbar ist es beispielsweise, Nanopartikel beizumischen, die den Wundheilungsprozess positiv beeinflussen.

Weiterhin bevorzugt wird eine Ausführungsform des Multifunktionselements, bei der das chirurgische Instrument zur Erzeugung des Aerosols einen Verdampfer aufweist. Weiterhin kann vorgesehen sein, dass statt eines Verdampfers eine Ultraschallerzeugungseinrichtung zur Erzeugung des Aerosols vorgesehen ist. Alternativ dazu kann jedoch auch eine Prallfläche vorgesehen sein, auf die das Oxidationsmittel prallt, so dass es beim Abprallen von der Fläche zerstäubt wird. Auf diese Weise kann das Gas-Oxidationsmittel-Gemisch besonders einfach durch die Karbonisierungsverhinderungsvorrichtung bereitgestellt werden. Auch wird eine Ausführungsform eines Multifunktionselements bevorzugt, die sich dadurch auszeichnet, dass wenigstens eine Zweistoffzerstäubereinrichtung bzw. Zweistoffdüse vorgesehen ist. Diese kann innenmischend oder außenmischend ausgebildet sein. Durch die Zweistoffzerstäubungseinrichtung ist es auf einfache Art und Weise möglich, ein Gas-Oxidationsmittel-Gemisch zur Erzeugung eines Gas-Oxidationsmittel-Plasmas bereitzustellen.

Darüber hinaus wird eine Ausführungsform eines Multifunktionselements bevorzugt, die sich dadurch auszeichnet, dass das chirurgische Instrument eine Ummantelung aufweist, die im Bereich der Elektrode wenigstens eine Öffnung zur Verhinderung einer Gasembolie aufweist. Zumindest wird dadurch die Wahrscheinlichkeit für eine Gasembolie oder eine Emphysembildung bei einem Kontakt der Ummantelung mit dem Gewebe wesentlich reduziert. Schließlich wird eine Ausführungsform eines Multifunktionselements bevorzugt, das einen Fluidapplikator zur Unterspritzung von Gewebe mit einer Flüssigkeit aufweist. Dadurch kann mit dem Multifunktionselement sowohl eine Wasserstrahlbehandlung als auch ein Schneidvorgang oder eine Plasma-Koagulation durchgeführt werden, ohne dass ein Wechsel des chirurgischen Instruments erforderlich ist. Der Fluidapplikator kann darüber hinaus dazu dienen, den OP-Situs zu reinigen. Vorzugweise wird dem Fluidapplikator das Oxidationsmittel der Karbonisierungsverhinderungsvorrichtung zur Unterspritzung von Gewebe zugeführt. Das Oxidationsmittel liegt dann vorzugsweise als Flüssigkeit vor, insbesondere handelt es sich bei dem Oxidationsmittel dann um Wasser.

Darüber hinaus kann vorgesehen sein, dass eine selbstansaugende Zweistoffzerstäubereinrichtung vorgesehen ist, die vorzugsweise durch die Anordnung des Gaszufuhrkanals und des Oxidationsmittelzufuhrkanals geschaffen wird und die eine zusätzliche Pumpe für die Zufuhr des Oxidationsmittels überflüssig macht.

Durch das Bereitstellen eines Gas-Oxidationsmittel-Gemisches zur Erzeugung eines Gas-Oxidationsmittel-Plasmas kann eine Karbonisierung des Gewebes wesentlich vermindert werden, da der entstehende Kohlenstoff durch das Oxidationsmittel oxidiert wird. Darüber hinaus findet gleichzeitig eine Kühlung der Oberfläche des Gewebes durch das Oxidationsmittel statt. Besonders bevorzugt wird dabei ein Oxidationsmittel, welches flüssig oder gasförmig ist. Es kann jedoch auch vorgesehen sein, dass das Oxidationsmittel als Aerosol vorliegt. In diesem Fall weist das chirurgische Instrument vorzugsweise eine entsprechende Vorrichtung zur Erzeugung des Aerosols auf. Das Oxidationsmittel muss geeignet sein, um Kohlenstoff zu oxidieren, was beispielsweise auf Wasser zutrifft. Als Gas wird vorzugsweise ein Inertgas, besonders bevorzugt Argon eingesetzt. Durch die Verwendung eines Multifunktionselements mit einer Karbonisierungsverhinderungsvorrichtung wird ein Gas-Oxidationsmittel-Gemisch zur Erzeugung eines Gas-Oxidationsmittel-Plasmas bereitgestellt, wodurch eine Karbonisierung des Gewebes effektiv vermindert wird.

Die Erfindung wird im Folgenden anhand der Zeichnung näher erläutert. Es zeigen:
Figur 1 eine schematische perspektivische Darstellung eines ersten chirurgischen Instruments;
Figur 2 eine schematische perspektivische Darstellung eines zweiten chirurgischen Instruments;
Figur 3 eine schematische perspektivische Darstellung eines dritten chirurgischen Instruments;
Figur 4 eine schematische perspektivische Darstellung eines vierten chirurgischen Instruments;
Figur 5 eine schematische Schnittdarstellung einer ersten Ausführungsform eines Multifunktionselements;
Figur 6 eine schematische Schnittdarstellung einer zweiten Ausführungsform eines Multifunktionselements;
Figur 7 eine schematische Schnittdarstellung einer dritten Ausführungsform eines Multifunktionselements;
Figur 8 eine perspektivische Darstellung einer vierten Ausführungsform eines Multifunktionselements;
Figur 9 eine schematische Darstellung einer Elektrode und einer Zweistoffdüse;
Figur 10 eine Draufsicht auf einen Austrittsbereich eines chirurgischen Instruments, und
Figur 11 eine schematische Schnittdarstellung einer Ausführungsform eines chirurgischen Instruments mit Venturi-Düse.

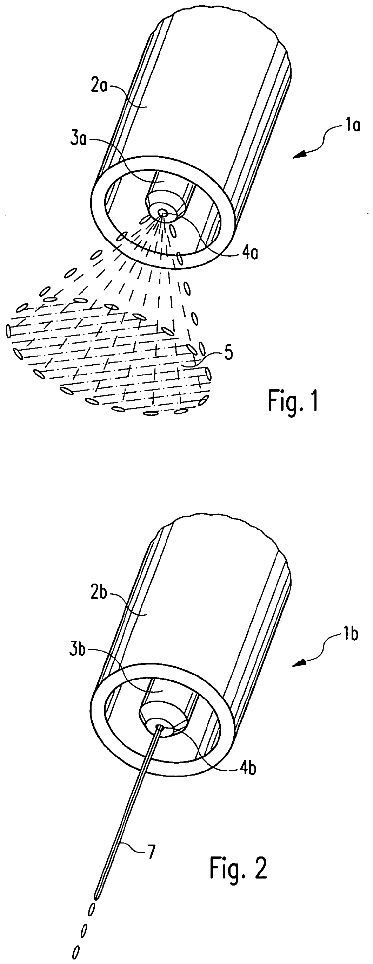

Figur 1 zeigt eine schematische perspektivische Darstellung eines ersten chirurgischen Instruments 1a. Es weist eine Ummantelung 2a auf, die eine Elektrode 3a umgibt, die an einen Hochfrequenzgenerator angeschlossen ist, der einen hochfrequenten Strom an die Elektrode 3a liefert. Die Elektrode 3a dient gleichzeitig als Zufuhrkanal für eine Flüssigkeitskomponente, die im Bereich einer zentral angeordneten Austrittsöffnung 4a aus der Elektrode 3a austritt.

Das chirurgische Instrument 3a gemäß Figur 1 ermöglicht es beispielsweise für eine endoskopische Submukosadissektion (ESD), das Gewebe mittels dem aus der Elektrode 3a austretenden Wasserstrahl zu unterspritzen und die Mukosa somit von der Muskularis zu trennen. Anschließend kann eine Inzision bzw. Dissektion des unterspritzten Gewebes erfolgen.

Figur 1 macht deutlich, dass die Flüssigkeitskomponente in Form eines aufgeweiteten, insbesondere kegelförmigen turbulenten Wasserstrahls 5 aus der Elektrode 3 austritt. Dieser kann im Übrigen nicht nur zum Unterspritzen der Mukosa, sondern auch zum Spülen des OP-Situs eingesetzt werden, um dem Operateur eine freie Sicht auf das Operationsfeld zu gewährleisten. Figur 2 zeigt eine schematische perspektivische Darstellung eines zweiten chirurgischen Instruments 1b mit einer Ummantelung 2b und einer darin angeordneten Elektrode 3b. Diese weist wiederum einen Zufuhrkanal für eine Flüssigkeitskomponente auf, wobei der Zufuhrkanal mit einer zentral angeordneten Austrittsöffnung 4b versehen ist, aus der die Flüssigkeitskomponente in Form eines laminaren Strahls 7 austritt, der vorzugsweise zur nadellosen Injektion einer Flüssigkeit, insbesondere von Wasser, in die Submukose als Schutzkissen bei der Dissektion der Mukosa dient.

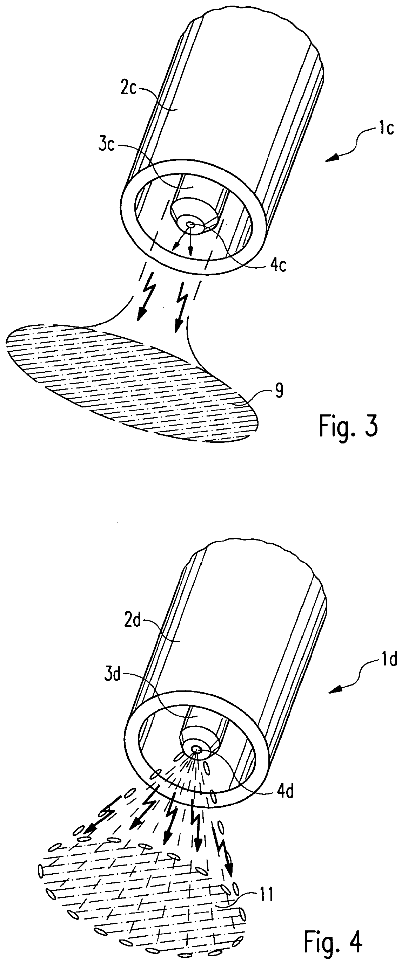

Es wird deutlich, dass sich das chirurgische Instrument 3b von dem in Figur 1 dargestellten chirurgischen Instrument 3a lediglich in der Form des austretenden Flüssigkeitsstrahls unterscheidet. Diese kann durch die Form des Austrittsbereichs 4a bzw. 4b verändert werden und an die Beschaffenheit des jeweils zu behandelnden Gewebes entsprechend angepasst sein. Figur 3 zeigt eine schematische perspektivische Darstellung eines dritten chirurgischen Instruments 1c mit einer Ummantelung 2c und einer darin angeordneten Elektrode 3c, die innen und/oder außen von Edelgas, insbesondere von Argon, umströmt wird. Ein derartiges chirurgisches Instrument 1c wird bei der Plasma-Koagulation, insbesondere bei der Argon-Plasma-Koagulation eingesetzt. Die Elektrode 3c ist wiederum an einen HF-Generator angeschlossen, der einen hochfrequenten Strom an die Elektrode liefert.

Figur 3 macht deutlich, dass das Edelgas im Bereich einer Austrittsöffnung 4c der Elektrode 3c durch das hochfrequente elektrische Wechselfeld gezündet wird, so dass zwischen der Elektrode 3c des chirurgischen Instruments 1c und einer hier nicht dargestellten Gewebeschicht ein Edelgas-Plasma 9 erzeugt wird.

Figur 4 zeigt eine schematische perspektivische Darstellung eines vierten chirurgischen Instruments 1d mit einer Ummantelung 2d und einer darin angeordneten Elektrode 3d, die mit einem HF-Generator verbunden ist und einen Zufuhrkanal mit einer Austrittsöffnung 4d aufweist. Der Zufuhrkanal wird von einem Gas-Oxidationsmittel-Gemisch durchströmt, welches im Bereich der Austrittsöffnung 4d in Form eines kegelförmigen Strahls 11 austritt und durch das hochfrequente elektrische Wechselfeld zu einem Gas-Oxidationsmittel-Plasma gezündet wird.

Das chirurgische Instrument 1d weist somit eine Karbonisierungsverhinderungsvorrichtung auf, die ein Gas-Oxidationsmittel-Gemisch zur Erzeugung eines Gas-Oxidationsmittel-Plasmas bereitstellt, deren Vorteile später noch näher erläutert werden. Durch die Karbonisierungsverhinderungsvorrichtung wird eine Karbonisierung des zu behandelnden Gewebes und eine damit verbundene Rauch- und Rauchgasentwicklung in vorteilhafter Weise vermieden.

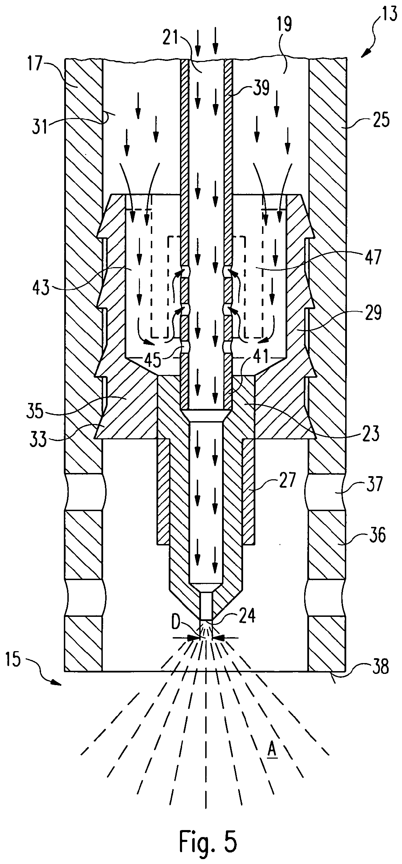

Figur 5 zeigt eine schematische Schnittdarstellung eines ersten Multifunktionselements 13 gemäß der Erfindung. Das Multifunktionselement 13 umfasst eine Karbonisierungsverhinderungsvorrichtung 15, die eine Karbonisierung von koaguliertem Gewebe vermindert, wobei eine Plasma-Koagulation mittels eines geeigneten chirurgischen Instruments 17 durchgeführt wird.

Wie im Folgenden noch näher erläutert wird, vereinigt das Multifunktionselement 13 die Funktionen der in den Figuren 1 bis 4 gezeigten chirurgischen Instrumente 1a bis 1d in vorteilhafter Weise. Somit ist es mit dem hier beschriebenen Multifunktionselement 13 möglich, eine Markierung und eine Elevation des zu behandelnden Gewebes durch dessen Unterspritzung vorzunehmen, anschließend eine Inzision/Dissektion des Gewebes durchzuführen und schließlich eine karbonisierungsarme Koagulation zu bewirken, während zusätzlich der OP-Situs mittels des Multifunktionselements 13 gereinigt werden kann.

Figur 5 macht deutlich, dass das chirurgische Instrument 17 eine Zuführung 19 für ein Gas aufweist, im Folgenden Gaszufuhrkanal 19 genannt, sowie eine Zuführung 21 für ein Oxidationsmittel, im Folgenden Oxidationsmittelzufuhrkanal 21 genannt. Weiterhin ist eine Elektrode 23 vorgesehen, der ein hochfrequenter Strom von einer hier nicht gezeigten HF-Spannungsquelle zugeführt wird. Die Elektrode 23 ist im Übrigen hohl ausgebildet und weist eine Austrittsöffnung 24 auf. Sie dient somit quasi als Verlängerung des Oxidationsmittelzufuhrkanals 21.

Der Gaszufuhrkanal 19, der Oxidationsmittelzufuhrkanal 21 und die Elektrode 23 sind gemäß Figur 5 von einer Ummantelung 25 umgeben, die vorzugsweise aus PTFE besteht und die mit einem hier nicht dargestellten HF-Chirurgiegerät verbunden ist.

Weiterhin ist ein Isolationsschutz 27 vorgesehen, der die Elektrode 23 zumindest bereichsweise koaxial umschließt. Ferner ist im Inneren der Ummantelung 25 eine Fixierhülse 29 vorgesehen, die an einer Innenwandung 31 der Ummantelung 25 mittels geeigneter Rastvorsprünge 33 befestigt ist und die mit ihrem distalen Bereich 35 die Elektrode 23 umschließt.

Das die Elektrode 23 umgebende distale Ende 36 der Ummantelung 25 weist im Übrigen laterale Öffnungen 37 auf, durch die das Gas-Oxidationsmittel-Gemisch entweichen kann, wobei eine Gasembolie und eine Emphysembildung vermieden werden sollen, wenn die Ummantelung 25 mit ihrer Stirnseite 38 auf dem zu behandelnden Gewebe aufliegen sollte.

Der Oxidationsmittelzufuhrkanal 21 ist in einem Rohr 39 ausgebildet, welches vorzugsweise aus rostfreiem Stahl, insbesondere aus V2A-Stahl besteht. Das Rohr 39 ist an einem nicht gezeigten proximalen Ende mit einer HF-Spannungsquelle verbunden und dient somit gleichzeitig als elektrischer Leiter, der die Elektrode 23 mit einem hochfrequenten Strom versorgt. Hierzu ist ein distales Ende 41 des Rohrs 39 mit der Elektrode 23 verbunden. Weiterhin ist das Rohr 39 mit einer hier nicht gezeigten Oxidationsmittelquelle verbunden, so dass ein Oxidationsmittel durch das Rohr 39 bzw. durch den Oxidationsmittelzufuhrkanal 21 und weiter durch die Elektrode 23 zu der Austrittsöffnung 24 der Elektrode 23 gelangen kann.

Zwischen der Fixierhülse 29 und dem Rohr 39 ist ein Ringraum 43 vorgesehen, in den das Gas aus dem Gaszufuhrkanal 19 geleitet wird. Weiterhin weist das Rohr 39 im Bereich des Ringraums 43 wenigstens eine, hier mehrere Öffnungen 45 auf, durch die das Gas aus dem Ringraum 43 in den Oxidationsmittelzufuhrkanal 21 strömen kann. In dem Ringraum 43 kann im Übrigen ein Diffusor 47 angeordnet sein, der in Figur 5 lediglich gestrichelt dargestellt ist.

Der Gaszufuhrkanal 19 und der Oxidationsmittelzufuhrkanal 21 des chirurgischen Instruments 17 bilden somit zusammen eine Zweistoffdüse, die vorliegend rein beispielhaft innenmischend ausgebildet ist, so dass also das Gas und das Oxidationsmittel getrennt einer Mischkammer zugeführt werden, wobei die Mischkammer bei der vorliegenden Ausführungsform des Multifunktionselements 13 durch den Oxidationsmittelzufuhrkanal 21 gebildet wird. Erst nach dem Mischen wird das Gas-Oxidationsmittel-Gemisch durch die Austrittsöffnung 24 nach außen in einen Austrittsbereich A geleitet, wobei die Austrittsöffnung 24 in der Elektrode 23 quasi eine Düse bildet. Die Austrittsöffnung 24 kann hierzu einen bestimmten Innendurchmesser D und eine geeignete Form aufweisen, um eine gewünschte Strahlbreite des ausgestoßenen Gas-Oxidationsmittel-Gemisches zu erzeugen. In Figur 5 ist die Austrittsöffnung 24 derart ausgebildet, dass der Strahl eine Kegelform aufweist.

Das Gas-Oxidationsmittel-Gemisch wird beim Austreten aus dem Oxidationsmittelzufuhrkanal 21 vorzugsweise zerstäubt, so dass das Oxidationsmittel bzw. das Gas-Oxidationsmittel-Gemisch in dem Austrittsbereich A als Aerosol vorliegt. Um eine Plasma-Koagulation durchzuführen, wird die Elektrode 23 bzw. das Multifunktionselement 13 in die Nähe des zu behandelnden Gewebes gebracht und das austretende zerstäubte Gas-Oxidationsmittel-Gemisch durch die Elektrode 23 bzw. den dort anliegenden hochfrequenten Strom gezündet, so dass ein leitfähiges Gas-Oxidationsmittel-Plasma erzeugt wird, durch das der hochfrequente Strom von der Elektrode 23 zu dem Gewebe fließen kann, um dort eine Koagulation zu bewirken.

Das oben beschriebene Multifunktionselement 13 kann somit unter anderem in vorteilhafter Weise für die Plasma-Koagulation, insbesondere für die Argon-Plasma-Koagulation eingesetzt werden. Das Multifunktionselement 13 weist eine Karbonisierungsverhinderungsvorrichtung 15 auf, die eine Karbonisierung des Gewebes während der Plasma-Koagulation nahezu vollständig vermeidet. Als Gas wird vorzugsweise Argon verwendet, welches durch den Gaszufuhrkanal 19, den Ringraum 43 und die Öffnungen 45 dem Oxidationsmittel zugeführt wird. Als Oxidationsmittel kann jeder Stoff dienen, der geeignet ist, Kohlenstoff zu oxidieren. Vorzugsweise wird jedoch Wasser als Oxidationsmittel eingesetzt, welches den bei der Plasma-Koagulation entstehenden Kohlenstoff oxidiert.

Das Oxidationsmittel kann im Übrigen flüssig oder gasförmig in den Oxidationsmittelzufuhrkanal 21 geleitet werden. Denkbar ist es auch, dass das Oxidationsmittel ein Feststoff ist. Falls das Oxidationsmittel in flüssiger Form in den Oxidationsmittelzufuhrkanal 21 geleitet wird, ist vorzugsweise vorgesehen, dass das Oxidationsmittel durch geeignete Mittel in ein Aerosol überführt wird. Auch kann der Eintrag des Oxidationsmittels in der jeweiligen gasförmigen Substanz erfolgen, wobei das gasförmige Oxidationsmittel vorher beispielsweise mittels eines Verdampfers erzeugt wurde.

In der Ausführungsform gemäß Fig. 5 ist beispielsweise vorgesehen, dass das Oxidationsmittel in flüssiger Form durch den Oxidationsmittelzufuhrkanal 21 geleitet wird und mit dem Gas aus dem Gaszufuhrkanal 19 versetzt wird. Das Gas-Oxidationsmittel-Gemisch wird anschließend der Austrittsöffnung 24 der Elektrode 23 zugeführt. Auf diese Weise wird das Gas-Oxidationsmittel-Gemisch zerstäubt, so dass es nach dem Austreten aus der Elektrode 23 als Aerosol vorliegt, wo es durch den HF-Strom zu einem Plasma gezündet wird. Es liegt somit ein Gas-Oxidationsmittel-Plasma vor.

Figur 5 macht deutlich, dass durch den Oxidationsmittelzufuhrkanal 21 nicht nur ein Oxidationsmittel-Gasgemisch geleitet werden kann, sondern dieser auch zur Zuführung einer Injektionsflüssigkeit zum Unterspritzen von Gewebe dienen kann. Dabei kann vorgesehen sein, dass das Oxidationsmittel als Injektionsflüssigkeit dient.

Durch den Oxidationsmittelkanal 21 weist das Multifunktionselement 13 folglich einen Wasserapplikator auf, der eine Unterspritzung von Gewebe zur Erzeugung eines Flüssigkeitskissens unter dem zu ablatierenden Gewebeareal bewirken kann. Der Wasserapplikator kann jedoch gleichzeitig dazu dienen, den OP-Situs bei auftretenden Blutungen zu reinigen, um so ein freies Sichtfeld für den Operateur zu gewährleisten. Das in Figur 5 gezeigte Multifunktionselement 13 kann damit im Wesentlichen drei unterschiedliche chirurgische/therapeutische Eingriffe realisieren. Einerseits ist die Realisierung eines Flüssigkeitskissens in der Submuskosa-Schicht der Schleimhaut zum Zweck des Aufbaus eines Thermoschutzkissens möglich. In diesem Fall wird die Gaszufuhr durch den Gaszufuhrkanal 19 vorzugsweise abgeschaltet, sodass ausschließlich das Oxidationsmittel, insbesondere Wasser durch den Oxidationsmittelzufuhrkanal 21 gleitet wird. Weiterhin kann das Multifunktionselement 13 zum Schneiden von Gewebe mittels der Elektrode 23 eingesetzt werden und eine Plasma-Koagulation durchgeführt werden. Hierzu wird durch den Gaszufuhrkanal 19 vorzugsweise ein Edelgas, insbesondere Argon in den Austrittsbereich A der Elektrode 23 geleitet, wobei der an der Elektrode 23 anliegende hochfrequente Strom ein Plasma zwischen der Elektrode und dem Gewebe zündet.

Schließlich kann das Multifunktionselement 13 mittels einer Karbonisierungsverhinderungsvorrichtung 15 in vorteilhafter Weise ein Aerosolplasma erzeugen. Dadurch vermindert das Multifunktionselement 13 eine Karbonisierung des Gewebes während der Plasma-Koagulation und eine damit verbundene Rauch- und Rauchgasentwicklung. Dadurch werden zum einen post-operative Probleme des Patienten ausgeräumt und zum anderen wird eine Verminderung der Sichtverhältnisse während der Operation vermieden, so dass die Karbonisierungsverhinderungsvorrichtung 15 des Multifunktionselements 13 sowohl für den Patienten als auch für den Operateur wesentliche Vorteile mit sich bringt. Schließlich kann auch vorgesehen sein, dass der Oxidationsmittelzufuhrkanal 21 zusätzlich als Zufuhrkanal für ein Spülmittel dient, welches zur Reinigung des OP-Situs dient.

Insgesamt zeigt sich somit, dass das Multifunktionselement 13 der hier beanspruchten Art im Wesentlichen drei chirurgisch/therapeutische Eingriffe ermöglicht, für die üblicherweise jeweils ein separates Instrument notwendig wäre. Die unterschiedlichen Funktionen des Multifunktionselements 13 können darüber hinaus vorteilhaft mit den optimierten Einstellparametern, möglichst unabhängig voneinander, beispielsweise über Fußschalter oder Instrumentenhandgriffe, aktiviert werden.

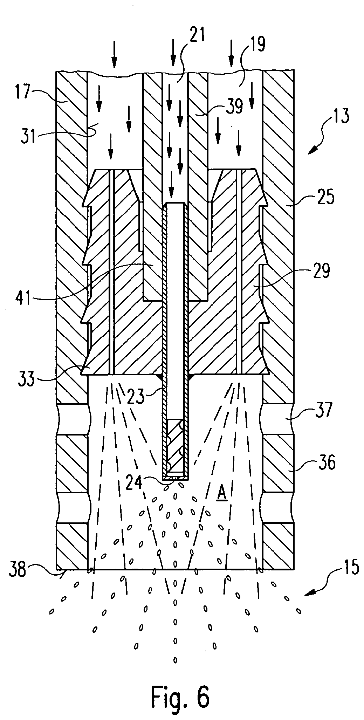

Figur 6 zeigt eine schematische Schnittdarstellung einer zweiten Ausführungsform eines Multifunktionselements 13 mit einer außenmischenden Zweistoffdüse unter Verwendung einer vorzugsweise spiralförmig ausgebildeten Fixierhülse 29 für die Zwei-Stoffflüssigkeitszerstäubung. Gleiche Teile sind mit gleichen Bezugszeichen versehen, sodass insofern auf die Beschreibung zu Figur 1 verwiesen wird, um Wiederholungen zu vermeiden. Das chirurgische Instrument 17 gemäß Figur 6 weist wiederum einen Gaszufuhrkanal 19 auf, der von einem Oxidationsmittelzufuhrkanal 21 umgeben ist, wobei die beiden Zufuhrkanäle in der Ummantelung 25 ausgebildet sind. Es versteht sich, dass die Anordnung des Oxidationsmittelzufuhrkanals und des Gaszufuhrkanals rein beispielhaft ist. Denkbar ist es auch, dass der Gaszufuhrkanal von dem Oxidationsmittelzufuhrkanal umgeben ist.

Entsprechend der Ausführungsform gemäß Figur 5 ist, wie oben bereits erläutert wurde, die Fixierhülse 29 vorgesehen, die koaxial in der Ummantelung 25 angeordnet ist und mit geeigneten Rastvorsprüngen 33 an der Innenwandung 31 der Ummantelung 25 befestigt ist. In der Ummantelung 25 sind wiederum seitliche Öffnungen 37 zur Verhinderung einer Gasembolie vorgesehen. Weiterhin ist die Elektrode 23 im Wesentlichen zentrisch in der Fixierhülse 29 gelagert und mit dem Rohr 39 verbunden, welches als Oxidationsmittelzufuhrkanal 21 dient. Die Elektrode 23 ist wiederum hohl ausgebildet und dient quasi als Verlängerung des Oxidationsmittelzufuhrkanals 21. Das distale Ende der Elektrode 23 weist auch bei der Ausführungsform gemäß Figur 6 eine Austrittsöffnung 24 auf, die als Düse mit einem geeigneten Durchmesser und einer geeigneten Form ausgebildet ist, so dass das in dem Oxidationsmittelzufuhrkanal 21 geführte Oxidationsmittel beim Austreten aus dem Kanal zerstäubt wird.

Entgegen der in Figur 5 gezeigten Ausführungsform des Multifunktionselements 13 ist in Figur 6 vorgesehen, dass die Zweistoffdüse, die durch den Gaszufuhrkanal 19 und den Oxidationsmittelzufuhrkanal 21 gebildet wird, außenmischend ausgebildet ist. Das Gas und das Oxidationsmittel werden also nicht einer gemeinsamen Mischkammer zugeführt und dann zerstäubt, sondern das Gas und das Oxidationsmittel werden in zwei getrennten Kanälen nach außen geleitet und bilden erst nach dem Austreten aus ihren jeweiligen Zufuhrkanälen 19 und 21 ein Gas-Oxidationsmittel-Gemisch in dem Austrittsbereich A. Hierzu ist in der Fixierhülse 29 wenigstens eine axiale Durchgangsbohrung 49 vorgesehen, welche den Gaszufuhrkanal 19 mit dem Austrittsbereich A verbindet, in den die Elektrode 23 hineinragt.

In Figur 6 sind in der Schnittdarstellung rein beispielhaft zwei Durchgangsbohrungen 49 vorgesehen. Es versteht sich, dass mehr als zwei Bohrungen vorgesehen sein können. Denkbar ist es jedoch auch, einen Ringraum oder dergleichen vorzusehen bzw. die Fixierhülse 29 zweiteilig auszubilden, sodass das Gas durch den Ringraum in den Austrittsbereich A gelangt.

Somit wird das Gas-Oxidationsmittel-Gemisch erst im Austrittsbereich A und nicht schon im Oxidationsmittelzufuhrkanal 21 gemäß Figur 5 bereitgestellt. Es kann auch vorgesehen sein, dass das Gas und das Oxidationsmittel im Austrittsbereich A am distalen Ende der Elektrode 23 so zusammenwirken, dass durch das Aufeinandertreffen des Gases und des Oxidationsmittels ein Zerstäuben des Oxidationsmittels herbeigeführt wird. Auf eine Zerstäuberdüse kann dann verzichtet werden.

Auch bei der Ausführungsform gemäß Figur 6 kann das Oxidationsmittel flüssig oder gasförmig sein. Beispielsweise ist es denkbar das Oxidationsmittel schon gasförmig durch den Oxidationsmittelzufuhrkanal 21 zu leiten. Vorzugsweise liegt das Oxidationsmittel im Austrittsbereich A jedoch als Aerosol vor. Um ein Aerosol zu erzeugen, weist das chirurgische Instrument 17 vorzugsweise einen Verdampfer oder Erhitzer auf. Weiterhin kann ein Aerosol durch eine Ultraschallerzeugungseinrichtung erzeugt werden. Denkbar ist jedoch auch der Einsatz einer Prallfläche, an der das Oxidationsmittel abprallt und zerstäubt wird.

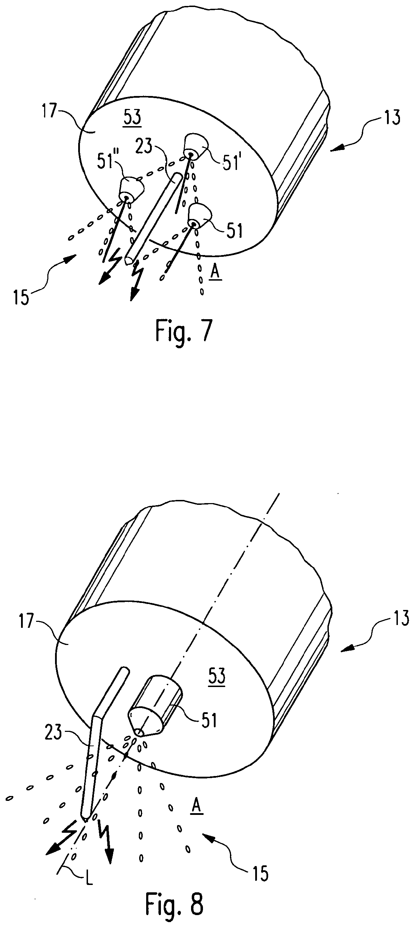

Figur 7 zeigt eine dritte Ausführungsform eines Multifunktionselements 13, wobei das chirurgische Instrument 17 eine zentrisch angeordnete stabförmige Elektrode 23 aufweist, die aus dem chirurgischen Instrument 17 herausragt.

Um die Elektrode 23 herum sind drei nicht näher gezeigte Zweistoffdüsen vorgesehen, die drei Austrittsöffnungen 51, 5' und 51" aufweisen. Beispielsweise kann das chirurgische Instrument 17 wie in Figur 5 oder Figur 6 dargestellt ausgebildet sein, wobei statt einer Zweistoffdüse insgesamt drei innenmischende oder außenmischende Zweistoffdüsen vorgesehen sind. Aus den Austrittsöffnungen 51, 5' und 51" strömt dann das Gas-Oxidationsmittel-Gemisch oder das Oxidationsmittel heraus. Die Austrittsöffnungen 51, 5' und 51" sind vorzugsweise so ausgebildet, dass das Gas-Oxidationsmittel-Gemisch zerstäubt wird, sodass es in Form eines Aerosols in dem Austrittsbereich A vorliegt. Der Grundkörper 53 des chirurgischen Instruments 17 ist im Übrigen vorzugsweise elektrisch isolierend ausgebildet.

Figur 8 zeigt eine perspektivische Darstellung einer vierten Ausführungsform eines Multifunktionselements 13. Gleiche Teile sind mit gleichen Bezugszeichen versehen, sodass insofern auf die Beschreibung zu den vorangegangenen Figuren verwiesen wird, um Wiederholungen zu vermeiden.

In Figur 8 ist die Elektrode 23 exzentrisch bezüglich des chirurgischen Instruments 17 angeordnet und ragt in den Austrittsbereich A hinein. Eine Austrittsöffnung 51 einer hier nicht näher dargestellten Zweistoffdüse ist hingegen zentrisch bezüglich des chirurgischen Instruments 17 angeordnet. Die Zweistoffdüse kann auch bei dieser Ausführungsform innen- oder außenmischend ausgebildet sein. Beispielsweise ist es denkbar, dass bei einer innenmischenden Zweistoffdüse gemäß der Ausführungsform gemäß Figur 5 ein Gas-Oxidationsgemisch aus der Austrittsöffnung 51 strömt, während bei einer außenmischenden Zweistoffdüse aus der Austrittsöffnung beispielsweise das Gas und aus der Austrittsöffnung 51 das Oxidationsmittel strömt, sodass erst im Austrittsbereich A das Gas-Oxidationsmittel-Gemisch bereitgestellt wird.

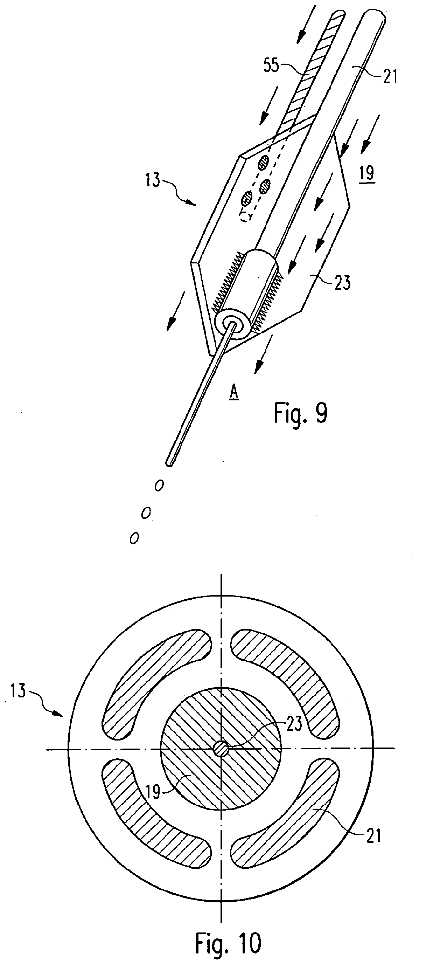

Figur 8 macht noch deutlich, dass die Elektrode 23 derart abgewinkelt geformt oder gebogen ist, dass sie von ihrer exzentrischen Austrittsposition aus in den Bereich einer Längsachse L des Multifunktionselements 13 bzw. der Austrittsöffnung 51 ragt. Der Grundkörper 53 des chirurgischen Instruments 17 ist im Übrigen vorzugsweise elektrisch isolierend ausgebildet. Figur 9 zeigt eine schematische Darstellung einer Ausführungsform einer Zweistoffdüse eines Multifunktionselements 13 mit einer Elektrode 23. Gleiche Teile sind mit gleichen Bezugszeichen versehen, sodass insofern auf die Beschreibung zu den vorangegangenen Figuren verwiesen wird, um Wiederholungen zu vermeiden.

Die Zweistoffdüse gemäß Figur 9 ist außenmischend ausgebildet. Die Elektrode 23 ist darüber hinaus als Metallplatte mit einer elektrischen Zuleitung 55 ausgebildet, die mit einer nicht gezeigten HF-Spannungsquelle verbunden ist, welche einen hochfrequenten Strom an die Elektrode 23 liefert. Die Metallplatte ist von einem Gaszufuhrkanal 19 umgeben, sodass das Gas an der Metallplatte vorbeiströmt. Der Oxidationsmittelzufuhrkanal 21 ist im Wesentlichen parallel an der Elektrode 23, also an der Metallplatte befestigt und erzeugt einen laminaren Strahl, sofern das Oxidationsmittel eine Flüssigkeit ist. Im Austrittsbereich A wird dann das Gas-Oxidationsmittel-Gemisch zur Erzeugung eines Gas-Oxidationsmittel-Plasmas bereitgestellt.

Figur 10 zeigt eine Draufsicht auf ein Multifunktionselement 13. Gleiche Teile sind mit gleichen Bezugszeichen versehen, sodass insofern auf die Beschreibung zu den vorangegangenen Figuren verwiesen wird, um Wiederholungen zu vermeiden. In Figur 10 ist ein außenmischendes Zweistoffdüsensystem dargestellt, wobei die Elektrode 23 zentral angeordnet ist und von dem Gaszufuhrkanal 19 umgeben ist. Koaxial und symmetrisch zu dem Gaszufuhrkanal 19 sind vier nierenförmige Oxidationsmittelzufuhrkanäle vorgesehen, die beispielsweise in der Ummantelung 25 oder die in einer Fixierhülse angeordnet sein können. Koaxial zu der Elektrode 1 ist darüber hinaus ein Gaszufuhrkanal 19 vorgesehen.

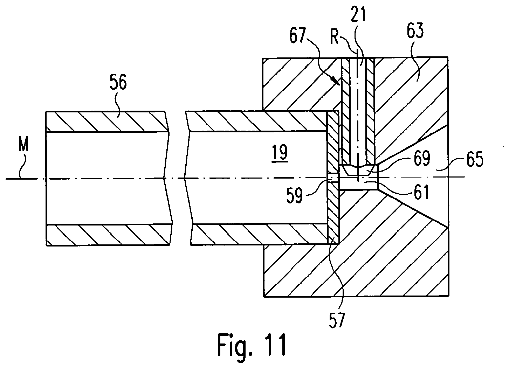

Figur 11 zeigt eine weitere Ausführungsform der Erfindung, bei der ein Gas-Oxidationsmittel-Gemisch bzw. ein Aerosol oder Gas-Oxidationsmittel-Plasma durch das Prinzip einer (Gas)Strahlpumpe, d.h. durch das Venturiprinzip erzeugt wird, wobei ein Unterdruck durch eine Verengung eines Zufuhrkanals erzeugt wird. Derartige Strahlpumpen sind grundsätzlich bekannt. Das Grundprinzip derartiger Pumpen besteht darin, dass aus einer Düse ein flüssiger oder gasförmiger Strahl mit hoher Geschwindigkeit austritt, der Flüssigkeit, Gas oder Feststoff aus seiner Umgebung mitreißt und beschleunigt.

Entsprechend kann gemäß der vorliegenden Erfindung ein eine Wandung 56 aufweisender Gaszufuhrkanal 19 für ein Gas, insbesondere für Argon vorgesehen sein. Das Gas strömt vorzugsweise durch eine am distalen Ende des Gaszufuhrkanals 19 angeordneten Blende 57 und insbesondere durch eine zentrische Austrittsöffnung 59 in der Blende 57 in einen zylinderförmigen Mischbereich 61 eines Aufsatzes 63, wobei der Aufsatz 63 am distalen Ende des Gaszufuhrkanals 19 angeordnet ist. Der Aufsatz 63 kann einstückig mit dem Gaszufuhrkanal 19 bzw. mit dessen Wandung 56 ausgebildet sein. Denkbar ist es aber auch den Aufsatz 63 als separates Teil auszubilden und mit dem Gaszufuhrkanal 19 auf geeignete Weise, insbesondere durch Kleben, Löten oder dergleichen, mit dem Gaszufuhrkanal 19 zu verbinden. An den zylinderförmigen Mischbereich 61 schließt sich ein kegelstumpfförmiger Zerstäuberbereich 65 an, der ebenfalls in dem Aufsatz 63 zentrisch ausgebildet ist.

Die Figur 11 macht deutlich, dass sich der Gaszufuhrkanal 19, die Öffnung 59, der Mischbereich 61 sowie der Zerstäuberbereich 65 entlang einer Mittelachse M erstrecken und im Wesentlichen symmetrisch zu dieser hintereinander angeordnet sind. Quer zu der Mittelachse M erstreckt sich entlang einer radialen Achse R ein Oxidationsmittelzufuhrrohr 67, in dem ein Oxidationsmittelzufuhrkanal 21 vorgesehen ist. Das Oxidationsmittelzufuhrrohr 67 ist mit einer nicht gezeigten Oxidationsmittelquelle verbunden. Das Oxidationsmittelzufuhrrohr 67 ist in eine entsprechende sich radial entlang der Achse R erstreckende Bohrung in dem Aufsatz 63 eingesetzt oder integral mit dieser ausgebildet und mündet mit seinem Endabschnitt 69 in den distalen zylinderförmigen Mischbereich 61.

Das Funktionsprinzip der Ausführungsform nach Figur 11 ist nun wie folgt: an der Engstelle im zylindrischen Mischbereich 61 hinter der Öffnung 59 muss der statische Druck des Gases aus Energieerhaltungsgründen geringer sein als an den nicht verengten Stellen der Vorrichtung. Durch den Gasstrom in dem Gaszufuhrkanal 19 bzw. in dem Mischbereich 61 wird daher ein zu zerstäubendes Oxidationsmittel, insbesondere Wasser, aus dem Oxidationsmittelzufuhrkanal 21 durch den Unterdruck im Mischbereich 61 angesaugt und von dem Gasstrom mitgerissen. Es handelt sich somit um eine (außenmischende) selbstansaugende Zweistoffdüse bzw. um eine Venturidüse, die den Vorteil hat, dass eine separate Pumpe für die Zuführung des Oxidationsmittels entfallen kann. Vielmehr wird das Oxidationsmittel durch den Gasfluss selbsttätig in den Mischbereich 61 gesaugt. Der Gaszufuhrkanal 19 und der Oxidationsmittelzufuhrkanal 21 sind bei dieser Ausführungsform der Erfindung folglich in vorteilhafter Weise als selbstansaugende Zweistoffzerstäubereinrichtung oder als (außenmischende) Venturi-Düse ausgebildet. Ausgehend von dem Mischbereich 61 kann dann in dem Zerstäuberbereich 65 das gewünschte Gas-Oxidationsmittel-Gemisch, insbesondere als Aerosol vorliegen und durch eine geeignete Elektrode ein Gas-Oxidationsmittel-Plasma gezündet werden.

Das Gas-Oxidationsmittel-Gemisch der hier angesprochenen Art weist mindestens zwei Komponenten auf, wobei die eine Komponente ein Gas, insbesondere ein Edelgas wie beispielsweise Argon oder Helium und die andere Komponente ein Oxidationsmittel für Kohlenstoff ist. Das Oxidationsmittel kann dabei aus festen oder flüssigen Schwebeteilchen, z.B. kleinen Wassertröpfchen bestehen, die als Wassernebel vorliegen. Hierbei wird flüssiges Oxidationsmittel sehr fein zerstäubt, so dass sich seine Oberfläche stark vergrößert. Auf diese Weise wird die Verdampfung erheblich beschleunigt, so dass neben den flüssigen Oxidationsmitteltröpfchen ein erheblicher Anteil an Oxidationsmitteldampf vorliegt. Durch den hochfrequenten Wechselstrom können auch Oxidationsmittelmoleküle, insbesondere Wassermoleküle in der Gasphase zu einem Wasserdampfplasma-Gemisch ionisiert werden.

Die obigen Ausführungen machen deutlich, dass das Gas-Oxidationsmittel-Gemisch vorzugsweise ein Aerosol ist, welches also gasförmige Teilchen und fein zerstäubte Oxidationsmitteltröpfchen aufweist. Mit dem Aerosolplasma soll weitgehend eine Karbonisierung des Gewebes vermieden werden, wobei der Oxidationsmittelnebel, insbesondere der Wassernebel, d.h. die H20-Tröpfchen gleichzeitig als Oxidationsmittel für Kohlenstoff, als Kühlmittel für die Gewebeoberfläche und als Plasmamedium wirkt.

Weiterhin ist die erhebliche Verminderung der Karbonisierung direkt mit der Emissionsmenge von Ruß und Rauchgasen wie C02, CO, NO, NOx, SOx, organischen und biochemischen Molekülen verbunden, so dass die vorgeschlagene Vorrichtung eine deutliche Verringerung oben genannter Emissionen zur Folge hat und damit die Expositionsrisiken von Patient und OP-Personal senkt.

Außerdem wird mit der vorgeschlagenen Karbonisierungsverhinderungsvorrichtung eine homogene und gewebeschonende Koagulation und Devitalisierung erreicht, welche darauf abzielt, das Verfahren schonend vorzugsweise im Bereich der Onkochirurgie, aber auch anderen medizinischen Disziplinen, z.B. zur Tumorablation, insbesondere bei dünnwandigen und nervsensiblen Strukturen, in der Neurochirurgie, Urologie und als adhäsionsreduzierende Chirurgiemethode in der Gynäkologie und Visceralchirurgie sowohl offenchirurgisch als auch endoskopisch (starr und flexibel) einzusetzen.

Weiterhin kann wenigstens eine Zweistoffdüse vorgesehen sein, die innenmischend oder außenmischend ausgebildet sein kann. Darüber hinaus kann das chirurgische Instrument 17 zur Erzeugung eines Oxidationsmittel-Aerosols bzw. eines Gas-Oxidationsmittel-Aerosols über geeignete Mittel verfügen, beispielsweise über einen Verdampfer, einen Ultraschallerzeuger oder über eine Prallplatte. Das Oxidationsmittel kann dabei entweder vor oder nach der Zusammenführung mit dem Gas zerstäubt werden. Entscheidend ist lediglich, dass in flüssige Oxidationsmitteltröpfchen in dem Gas vorhanden sind, um die oben beschriebenen Vorteile zu bewirken. Die Karbonisierungsverhinderungsvorrichtung 15 des Multifunktionselements 13 vermindert damit effektiv eine Karbonisierung und eine Russ- und Rauchentwicklung. Weiterhin wird eine gleichmäßigere Verteilung der Plasmaenergie auf der Gewebeoberfläche erzielt.

Insgesamt zeigt sich, dass die vorliegende Erfindung ein Multifunktionselement 13 mit einer Karbonisierungsverhinderungsvorrichtung 15 schafft, mit dem in vorteilhafter Weise mehrere Eingriffe durchgeführt werden können, ohne dass ein Instrumentenwechsel erforderlich ist. Darüber hinaus sorgt die Karbonisierungsverhinderungsvorrichtung 15 des Multifunktionselements 13 für die Bereitstellung eines Gas-Oxidationsmittel-Gemisches zur Erzeugung eines Gas-Oxidationsmittel-Plasmas. Auf diese Weise ist eine Verminderung der Karbonisierung bei der Plasma-Koagulation möglich.

### Bezugszeichenliste 1a-d chirurgisches Instrument

- 2a-d: Ummantelung
- 3a-d: Elektrode
- 4a-d: Austrittsöffnung
- 5: Flüssigkeitsnebel
- 7: Laminarer Wasserstrahl
- 9: Edelgas-Plasma
- 11: Gas-Oxidationsmittel-Gemisch
- 13: Multifunktionselement
- 15: Karbonisierungsverhinderungsvorrichtung
- 17: Chirurgisches Instrument
- 19: Gaszufuhrkanal
- 21: Oxidationsmittelzufuhrkanal 23 Elektrode
- 24: Austrittsöffnung
- 25: Ummantelung
- 27: Isolationsschutz
- 29: Fixierhülse
- 31: Innenwandung
- 33: Rastvorsprünge
- 35: Distaler Bereich (Fixierhülse)
- 36: Distales Ende (Ummantelung)
- 37: Laterale Öffnung
- 38: Stirnseite
- 39: Rohr
- 41: Distales Ende (Rohr)
- 43: Ringraum
- 45: Öffnung
- 47: Diffusor
- 49: Durchgangsbohrung
- 51: Austrittsöffnung
- 51': Austrittsöffnung
- 51": Austrittsöffnung
- 53: Grundkörper
- 55: Elektrische Zuleitung
- 56: Wandung
- 57: Blende
- 59: Austrittsöffnung
- 61: Mischbereich
- 63: Aufsatz
- 65: Zerstäuberbereich
- 67: Oxidationsmittelzufuhrrohr
- 69: Endabschnitt
- A: Austrittsbereich
- D: Innendurchmesser
- L: Längsachse
- M: Mittelachse
- R: Radiale Achse

## Patentansprüche

1. Multifunktionselement (13), geeignet zur Durchführung von mindestens zwei chirurgisch/therapeutischen Eingriffen, nämlich das Unterspritzen und/oder Schneiden von Gewebe sowie die Koagulation von Gewebe, aufweisend:
- eine Plasmakoagulationseinrichtung,
- einen Fluidapplikator zum Schneiden und/oder Unterspritzen von Gewebe mit Wasser und
- eine Karbonisierungsverhinderungsvorrichtung (15) zum Verhindern der Karbonisierung von Gewebe bei einer Plasma-Koagulation mittels eines geeigneten chirurgischen Instruments (17),
wobei das chirurgische Instrument (17):
- einen in einem Rohr (39) ausgebildeten Oxidationsmittelzuführungskanal (21) zur Zuführung eines flüssigen Oxidationsmittels in Gestalt von Wasser,
- einen Gaszufuhrkanal (19) zur Zuführung von Argon und
- eine Elektrode (23) zur Erzeugung eines Plasmas aufweist, wobei durch die Karbonisierungsverhinderungsvorrichtung (15) ein Aerosol zur Erzeugung eines Argons-Wasser-Plasmas bereitgestellt wird, **dadurch gekennzeichnet dass** das Rohr (39) mehrere Öffnungen (45) aufweist, durch die das Argon in den Oxidationsmittelzufuhrkanal (21) strömen kann, so dass Argon und Wasser getrennt einer Mischkammer zugeführt werden, die durch den Oxidationsmittelzuführungskanal (21) gebildet ist.

2. Multifunktionselement nach Anspruch 1, **dadurch gekennzeichnet, dass** das chirurgische Instrument (17) zur Erzeugung des Aerosols eine Prallplatte aufweist.

## Claims

1. Multi-functional element (13)which is suitable for performing at least two surgical/therapeutical interventions, namely sucutaneous injection and cutting of tissue and coagulation of tissue, comprising:
- a plasma coagulation device,
- a fluid applicator for cutting and/or subcutaneous injection of tissue with water, and
- an anti-carbonisation device (15) for preventing the carbonisation of tissue during plasma-coagulation by means of a suitable surgical instrument (17),
wherein the surgical instrument (17) comprises:
- an oxidation agent supply channel (21) formed in a pipe (39) and intended for the supply of a liquid oxidation agent in the form of water,
- a gas supply channel (19) for the supply of argon, and
- an electrode (23) for generating a plasma,
wherein said anti-carbonisation device (15) provides an aerosol in order to generate an argon-water plasma,
**characterised in that** the pipe (39) has several openings (45) through which the argon can flow into the oxidation agent supply channel (21) so that argon and water are supplied separately to a mixing chamber formed by the oxidation agent supply channel (21).

2. Multi-functional element according to claim 1, **characterised in that** the surgical instrument (17) comprises a rebound plate for generating the aerosol.

## Revendications

1. Elément multifonctionnel (13) adapté à la réalisation d'au moins deux interventions chirurgicales/thérapeutiques, à savoir l'injection sous des tissus et/ou l'incision de tissus, ainsi qu'à la coagulation de tissus, présentant :
- un dispositif de coagulation par plasma,
- un applicateur de fluide destiné à inciser des tissus et/ou à injecter de l'eau sous des tissus, et
- un dispositif anticarbonisation (15) destiné à empêcher la carbonisation de tissus lors d'une coagulation par plasma au moyen d'un instrument chirurgical (17) approprié,
l'instrument chirurgical (17) présentant :
- une conduite d'alimentation en agent oxydant (21) réalisée dans un tube (39) et destinée à amener un agent oxydant liquide sous forme d'eau,
- une conduite d'alimentation en gaz (19) destinée à amener de l'argon, et
- une électrode (23) destinée à générer un plasma,
le dispositif anticarbonisation (15) fournissant un aérosol pour la génération d'un plasma argon-eau,
**caractérisé en ce que** le tube (39) présente plusieurs orifices (45) à travers lesquels l'argon peut s'écouler dans la conduite d'alimentation en agent oxydant (21), de manière à ce que l'argon et l'eau soient acheminés séparément à une chambre de mélange qui est formée par la conduite d'alimentation en agent oxydant (21).

2. Elément multifonctionnel selon la revendication 1, **caractérisé en ce que** l'instrument chirurgical (17) présente une plaque déflectrice pour la génération de l'aérosol.
